# EUROPEAN PATENT SPECIFICATION

(11) **EP 2 585 585 B1**
(45) Date of publication and mention of the grant of the patent: **12.08.2015**
(21) Application number: 11727171.8
(22) Date of filing: 22.06.2011
(51) Int. Cl.: C12N 5/00

(54) **METHOD FOR HARVESTING CELLS CULTURED IN 3D HYDROGEL MATRICES**
VERFAHREN ZUR GEWINNUNG VON ZELLEN AUS DREIDIMENSIONALEN HYDROGELMATRIX-KULTUREN
PROCÉDÉ DE RÉCOLTE DE CELLULES CULTIVÉES DANS DES MATRICES D'HYDROGEL 3D

(30) Priority: 22.06.2010 EP 10305667
(43) Date of publication of application: 01.05.2013
(73) Proprietor: UNIVERSITE DE ROUEN, 76130 Mont-Saint-Aignan (FR)
(72) Inventor: VANNIER, Jean Pierre, 76160 Saint Jacques Sur Darnetal (FR); DEMANGE, Elise, 76000 Rouen (FR)
(74) Representative: Cabinet Plasseraud
(86) International application number: PCT/EP2011/060467
(87) International publication number: WO 2011/161174

(56) References cited:
- GERECHT SHARON ET AL: "Hyaluronic acid hydrogen for controlled self-renewal and differentiation of human embryonic stem cells", PROCEEDINGS OF THE NATIONAL ACADEMY OF SCIENCES OF THE UNITED STATES OF AMERICA, vol. 104, no. 27, July 2007 (2007-07), pages 11298-11303, XP002602538, ISSN: 0027-8424
- FENG QI ET AL: "Expansion of engrafting human hematopoietic stem/progenitor cells in three-dimensional scaffolds with surface-immobilized fibronectin.", JOURNAL OF BIOMEDICAL MATERIALS RESEARCH. PART A 15 SEP 2006 LNKD- PUBMED:16739181, vol. 78, no. 4, 15 September 2006 (2006-09-15), pages 781-791, XP002602539, ISSN: 1549-3296
- DAWSON ET AL: "Biomaterials for stem cell differentiation", ADVANCED DRUG DELIVERY REVIEWS, ELSEVIER BV, AMSTERDAM, NL LNKD- DOI:10.1016/J.ADDR.2007.08.037, vol. 60, no. 2, 11 October 2007 (2007-10-11), pages 215-228, XP022388009, ISSN: 0169-409X
- SERBAN MONICA A ET AL: "Unit 10.14: Use of hyaluronan-derived hydrogels for three-dimensional cell culture and tumor xenografts", CURRENT PROTOCOLS IN CELL BIOLOGY, vol. Chapter 10, no. Sup. 40, September 2008 (2008-09), pages 10.14.1-10.14.21, XP002602540, EDITORIAL BOARD, JUAN S. BONIFACINO ... [ET AL.] ISSN: 1934-2616

## Description

### Related Patent Applications

The present application claims priority to European Patent Application No. EP 10 305 667 filed on June 22, 2010. The European Patent Application is incorporated herein by reference in its entirety.

### Background of the Invention

The majority of cell culture studies have been performed on 2-dimensional (2D) surfaces such as micro-well plates, tissue culture flasks, and Petri dishes because of the ease, convenience, and high cell viability of 2D culture. Although these conventional 2D cell culture systems have tremendously improved our understanding of basic cell biology, they have proved to be insufficient and unsuitable for new challenges in cell biology as well as for pharmaceutical assays. Indeed, 2D culture systems fall short of reproducing the complex and dynamic environments of the *in vivo* situation, which are known to affect cell morphology, growth rates, contact geometries, transport properties, and numerous other cellular functions.

Three-dimensional (3D) cell culture matrices, also called scaffolds, have been introduced to overcome 2D culture limitations. These matrices are porous substrates that can support cell growth, organization, and differentiation on or within their structure. It has been demonstrated that, in comparison to conventional cultures, cells in 3D cultures more closely resemble the *in vivo* situation with regard to cell shape and cellular environment. Architectural and material diversity is much greater on 3D matrices than on 2D substrates. A variety of fabrication processes and biomaterials have been developed or adapted to produce cellular supports with different physical appearance, porosity, permeability, mechanical characteristics, and nano-scale surface morphology to match the diversity of *in vivo* environments.

Thus, for example, a lot of efforts have focussed on exploring the use of natural substances related to the extracellular matrix (ECM) as biomaterials for cell scaffolds, since the behavior of normal and tumor cells is known to be directly conditioned by the ECM composition. Therefore, 3D matrices have been produced from crosslinked hyaluronic acid and/or collagen, which are the principal constituents of ECM. Other components of the extracellular matrix, such as fibronectin, laminin, elastin, keratin sulphate, chondroitin sulphate and keratin sulfate, have also been tested as cell scaffold biomaterials, as have chemical derivatives of these naturally-occurring macromolecules. Synthetic polymers have proved to be attractive alternatives to these nature-derived macromolecules, because they provide the ability to tailor shape and mechanical properties as well as degradation kinetics to suit a variety of applications.

If 3D cell culture has significantly progressed thanks to the development of different matrices and different culture media, there is still a need in the art for a satisfactory method for harvesting cells grown on such matrices, if desired. Indeed, commonly used harvesting procedures, such as protease treatment, subject the cells to harsh conditions which, at best, result in temporary damage of cell structures and functions.

### Summary of the Invention

The present invention generally relates to a method for harvesting cells grown in three dimensions using a hydrogel matrix. The method is fast, simple and easy to implement as well as efficient in terms of yield. Furthermore, in contrast to commonly used cell harvesting procedures, the present invention does not damage cells and provides cells that are healthy, viable and fully functioning.

More specifically, in one aspect, the present invention provides a method for harvesting cells grown on a 3D hydrogel matrix, said method comprising steps of: (a) submitting the 3D hydrogel matrix containing cells to a dilaceration to obtain a dilacerated hydrogel matrix; (b) incubating the dilacerated hydrogel matrix with an enzyme that specifically degrades at least one component of the 3D hydrogel matrix to obtain an enzymatically degraded hydrogel matrix; and (c) submitting the enzymatically degraded hydrogel matrix to a dilaceration to obtain a suspension comprising cells grown in the 3D hydrogel matrix and fragments of the 3D hydrogel matrix.

In certain embodiments, the step of incubating the dilacerated hydrogel matrix with an enzyme is performed in an incubator at 37°C, in a 5% CO₂ atmosphere, and under sterile conditions.

In certain embodiments, the enzyme specifically degrades at least one component of the 3D hydrogel matrix. Preferably, the enzyme specifically degrades the main component of the matrix.

In certain embodiments, the dilacerations of steps (a) and (b) are performed using a tissue dissociation or tissue homogenization instrument, and preferably an automated tissue dissociation or tissue homogenization instrument. Preferably, the dilacerations are performed under sterile conditions.

In certain embodiments, a harvesting method of the present invention further comprises steps of: (d) filtrating the suspension comprising cells grown on the 3D hydrogel matrix and fragments of the 3D hydrogel matrix obtained in step (c) using a filter; (e) washing the filter; and (f) collecting the filtrate, wherein the filtrate is a suspension of cells grown on the 3D hydrogel matrix. Optionally, a method of the invention may further comprise a step of: centrifugating the filtrate to obtain cells that were grown on the 3D hydrogel matrix.

The cells grown on the 3D hydrogel matrix may be of a single cell type. Alternatively, the cells grown on the 3D hydrogel matrix may comprise at least two different types of cells. Cells that may be grown on the 3D hydrogel matrix include stem cells, induced pluripotent stem cells, progenitor cells, differentiated cells, and any combination thereof.

The 3D hydrogel matrix in which the cells are cultured prior to harvesting, may be any hydrogel matrix suitable for 3D cell culture and may comprise naturally-occurring macromolecules, modified biopolymers and/or synthetic polymers. In certain embodiments, the 3D hydrogel matrix is a 3D crosslinked hyaluronan hydrogel. In such embodiments, the enzyme used in step (b) is hyaluronidase.

Preferably, prior to cell culture, the 3D crosslinked hyaluronan hydrogel is (i) lyophilized; and (ii) sterilized first by heating the hydrogel, and then by immersing the hydrogel in pure alcohol and submitting the immersed hydrogel to physical compression according to a method developed in the laboratory of the present inventors. This method is described in European patent application number EP 10 305 666 and European patent application number EP 11 305 333.

Also dislosed are cells that were grown on a 3D hydrogel matrix and harvested using a method described herein. The isolated cells may be of a single type *(i.e.,* constitute a homogeneous population) or may comprise at least two different cell types *(i.e.,* constitute a heterogeneous population). Immediately after harvesting, the cells harvested according to the present invention are healthy, viable and fully functioning.

Also disclosed is the use of cells grown on a 3D hydrogel matrix and harvested using a method described herein for cell transplantation or tissue engineering or any other application requiring the immediate availability of functioning cells.

These and other objects, advantages and features of the present invention will become apparent to those of ordinary skill in the art having read the following detailed description of the preferred embodiments.

### Brief Description of the Drawing

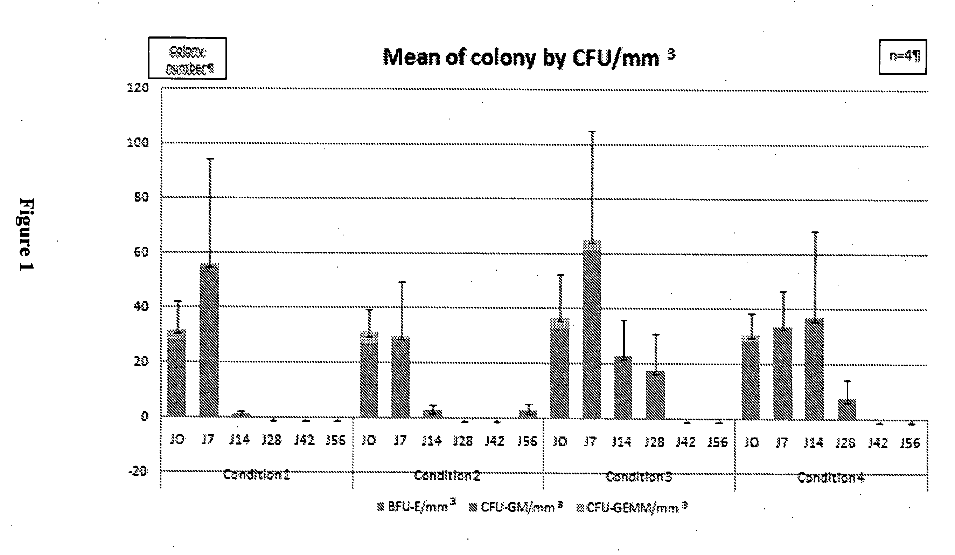

**Figure 1** is a graph showing the mean CFU values (determined as described in the Examples section below) for each sample of hematopoietic progenitor cells harvested from a set of three hydrogels, as a function of culture time, and for 4 different culture conditions: 50 ng/mL SCF (condition 1), 50 ng/mL SCF + 10 ng/mL of VEGF (condition 2), 50 ng/mL SCF + 300 ng/mL Ang-1 (condition 3), and 50 ng/mL SCF + 10 ng/mL of VEGF + 300 ng/mL Ang-1 (condition 4). The hematopoietic progenitor cells studied in these experiments stayed in the hydrogels during cell culture. The mean values are expressed in CFU/mm³ of hydrogel.

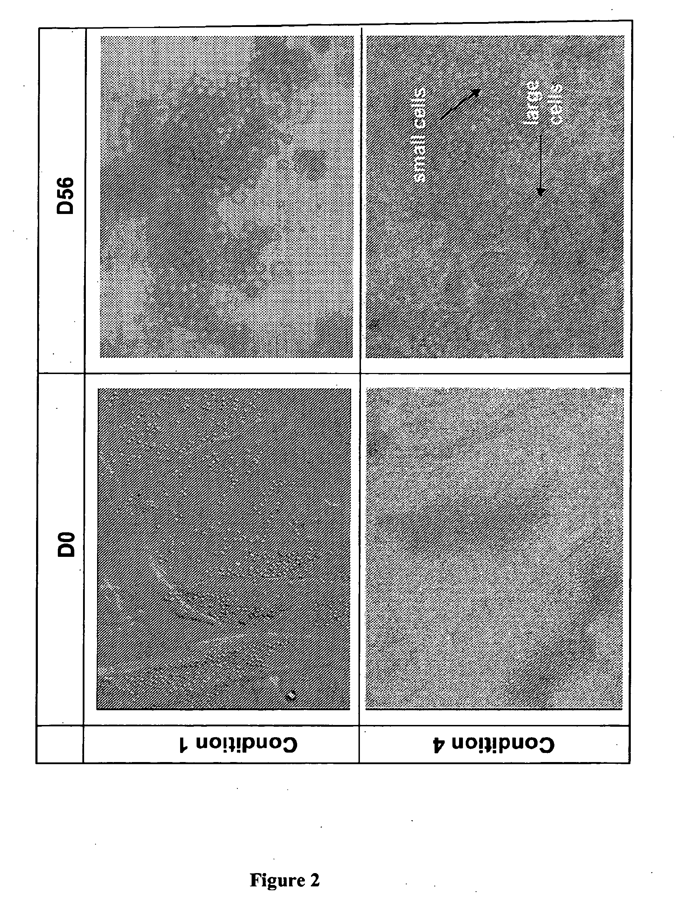

**Figure 2** is a set of four microscope pictures (x 100) of colonies of hematopoietic progenitor cells cultured in crosslinked hyaluronan hydrogels for 0 day (D0) or for 56 days (D56) under culture condition 1 (50 ng/mL SCF) or culture conditions 4 (50 ng/mL SCF, 10 ng/mL VEGF and 300 ng/mL Ang-1). The hematopoietic progenitor cells studied in these experiments stayed in the hydrogels during cell culture.

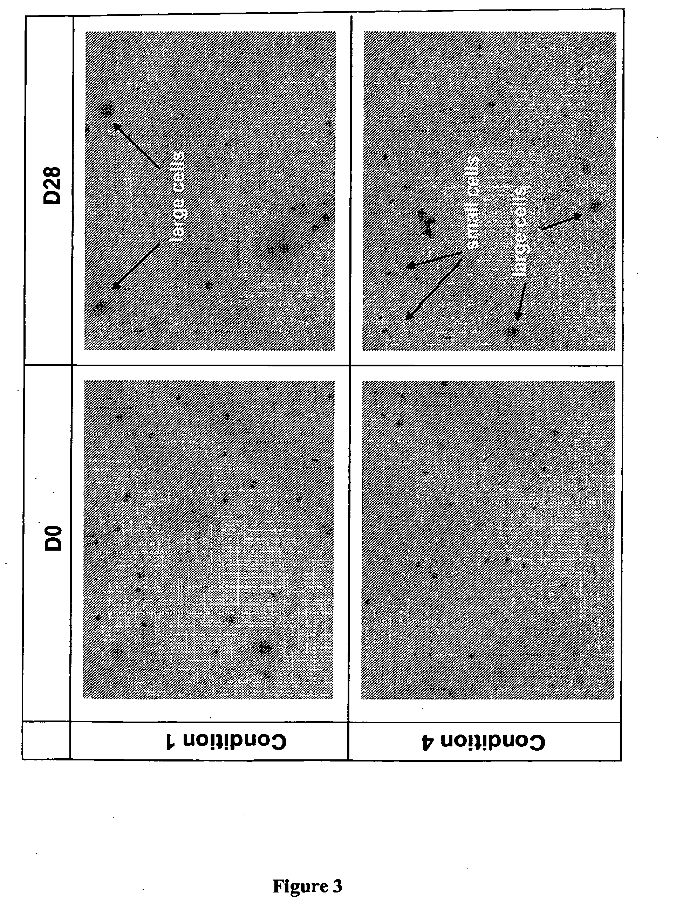

**Figure 3** is a set of four microscope pictures (x 100) of colonies of hematopoietic progenitor cells cultured in crosslinked hyaluronan hydrogels for 0 day (D0) or for 28 days (D56) under culture condition 1 (50 ng/mL SCF) or culture conditions 4 (50 ng/mL SCF, 10 ng/mL VEGF and 300 ng/mL Ang-1), The hematopoietic progenitor cells studied in these experiments stayed in the hydrogels during cell culture.

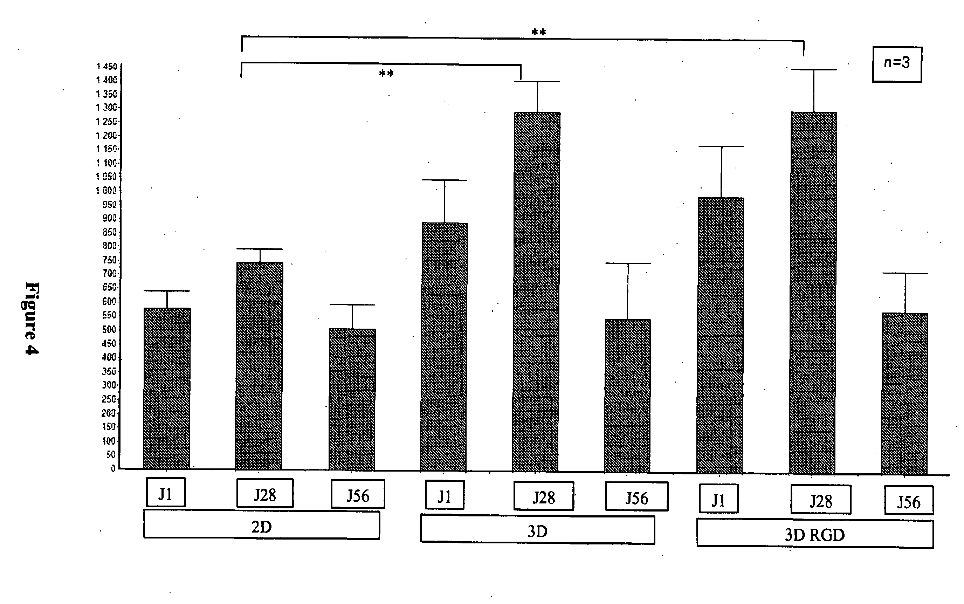

**Figure 4** is a graph showing the mean CFU values (determined as described in the Examples section below) for each sample of hematopoietic progenitor cells harvested from a set of three hydrogels, as a function of culture time, and for 3 different culture conditions: without hydrogel (negative control) or 2D culture, with hydrogel without coating (3D culture), hydrogel coated with 50 ng/mL RGD (Pronectin F plus(r)) (3D RGD culture. The hematopoietic progenitor cells were cultured using a culture medium (Stemspan) containing 50 ng/mL SCF + 0.1% of antibiotics. The hematopoietic cells studied in these experiments stayed in the hydrogels during cell culture. The mean values are expressed in global CFU.

### Definitions

Throughout the specification, several terms are employed that are defined in the following paragraphs.

As used herein, the term ***"hydrogel"*** has its art understood meaning and refers to a water-swellable polymeric matrix that can absorb water to form gels of varying elasticity. The term ***"matrix"*** refers to a 3D network of macromolecules held together by covalent and/or non-covalent cross-links. On replacement in aqueous environment, dry hydrogels swell to the extent allowed by the degree of cross-linking. The amount of water absorbed can be controlled by the macromolecule component used. A hydrogel can further enclose or comprise any number of biomolecules, pharmaceutically active agents, and/or biologically active agents.

As used herein, the term ***"differentiated cells**"* refers to cells specialized for a particular function and that do not have the ability to generate other kinds of cells. Examples of such cells include, but are not limited to, cardiomyocytes, neurons, skeletal muscle cells, hepatocytes, and the like.

As used herein, the term ***"stem cells**"* refers to relatively undifferentiated cells that have the capacity for sustained self-renewal as well as the potential to give rise to differentiated progeny *(i.e.,* to different types of specialized cells). The terms ***"embryonic stem cells**"* and ***"ES cells"*** are used herein interchangeably. They refer to stem cells derived from a group of cells called the inner cell mass, which is part of the early (4 to 5 days old) embryo called the blastocyst. ***"Human embryonic stem cells"*** or ***"hES cells**"* are embryonic stem cells of human origin that are generally derived from fertilized embryos that are less than one week old. *In vitro,* embryonic stem cells can proliferate indefinitely, a property that is not shared by adult stem cells.

The terms ***"progenitor cells"*** and ***"precursor cells"*** are used herein interchangeably. They refer to cells that occur in fetal or adult tissue and are partially specialized. These cells divide and give rise to differentiated cells. Progenitor or precursor cells belong to a transitory amplifying population of cells derived from stem cells. Compared to stem cells, they have a limited capacity for self-renewal and differentiation. Such capacity for self-renewal (or proliferation) is demonstrated by the expression of proliferation markers such as, for example, Ki-67 nuclear antigen. Moreover, since progenitor cells are committed to a particular differentiation process, progenitor cells also express specific markers.

The terms ***"induced pluripotent stem cells"*** and ***"iPS cells**"* are used herein interchangeably. They refer to a type of pluripotent stem cells artificially derived from non-pluripotent cells (e.g., adult somatic cells). Induced pluripotent stem cells are identical to embryonic stem cells in their ability to form any differentiated cell, but are not derived from an embryo. The terms ***"human induced pluripotent stem cells"*** and ***"human iPS cells"*** are used herein interchangeably. They refer to induced pluripotent stem cells of human origin. Typically, a human induced stem cell may be obtained through the induced expression of Oct3/4, Sox2, Klf4, and c-Myc genes in any adult somatic cell *(e.g.,* fibroblast). For example, human induced pluripotent cells may be obtained according to the protocol described by Takahashi et al. (Cell, 2007, 131: 861-872), by Yu et al. (Science, 2007, 318: 1917-1920) or by any other protocol in which one or the other agents used for reprogramming cells in these protocols are replaced by any gene or protein acting on or transferred to the somatic cells at the origin of the iPS lines. Basically, somatic cells are transfected with viral vectors, such as retroviruses, which comprise Oct3/4, Sox2, Klf4, and c-Myc genes.

The term ***"substantially homogeneous cell population",*** as used herein, refers to a population of cells wherein the majority (*e.g.,* at least about 90%, preferably at least about 95%, more preferably at least about 99%) of the total number of cells belong to a single cell type. The term ***"heterogeneous cell population",*** as used herein, refers to a population of cells comprising at least two cell types.

The terms ***"pharmaceutically active agent"*** and ***"therapeutically active agent"*** are used herein interchangeably. They refer to a substance, molecule, compound, agent, factor or composition that is effective in the treatment of a disease or a condition.

The term ***"biologically active agent"*** refers to a substance, molecule, compound, agent, factor or composition that affects (*e.g.,* modifies, prevents, inhibits, reverses, or enhances) a biological event or biological mechanism. Some biologically active agents may be pharmaceutically active agents.

As used herein, the term ***"effective amount"*** refers to any amount of a substance, molecule, agent, factor or composition that is sufficient to fulfil its intended purpose(s), e.g., a desired biological or medicinal response to a cell, tissue, system or subject.

The terms ***"approximately"*** and ***"about",*** as used herein in reference to a number, generally include numbers that fall within a range of 10% in either direction of the number (greater than or less than the number) unless otherwise stated or otherwise evident from the context (except where such number would exceed 100% of a possible value).

Other terms are defined in the following section when needed.

### Detailed Description of Certain Preferred Embodiments

As mentioned above, the present invention provides a method for harvesting cells grown in a 3D hydrogel matrix. In contrast to standard harvesting procedures, a method according to the invention does not subject cells to harsh conditions that are disruptive to cell structures and functions. Thus, cells harvested according to the invention are healthy, viable and fully functioning, and can therefore be used in a variety of applications such as cell transplantation and tissue engineering.

### I - Methods of Harvesting Cells Grown on 3D Hydrogel Matrices

A method according to the present invention generally comprises a combination of mechanical and enzymatic steps. More specifically, in a method of the invention, cells grown on a 3D hydrogel matrix are harvested by submitting the hydrogel to a first dilaceration step, by incubating the dilacerated hydrogel with an enzyme that degrades at least one component of the hydrogel, and by submitting the resulting slurry to a second dilaceration. The terms ***"harvesting", "retrieving**"* and ***"collecting"*** are used herein interchangeably. They refer to a process in which cells are detached or isolated from the hydrogel matrix in which they were grown.

### 1 - Harvesting method

### a - Dilaceration

The first step of a method according to the invention includes dilacerating a 3D hydrogel matrix in which cells have been cultured. The terms ***"dilaceration", "disintegration"*** and ***"dissociation**"* are used herein interchangeably. They refer to a process in which the hydrogel is mechanically torn into pieces. In the practice of the present invention, dilaceration is preferably carried out under sterile conditions. In addition, the hydrogel to be dilacerated is preferably immersed in a culture medium at room temperature *(i.e.,* between 18 and 25°C) during the dissociation process.

Dilaceration of a 3D hydrogel matrix may be performed using any suitable means. In particular, it may be advantageously performed using means generally intended for the dissociation or homogenization of biological tissues (*e.g.,* animal or human tissues). Dilaceration may be achieved *via* a single mechanism or by a combination of different mechanisms. Thus, dilaceration may be obtained through mechanical shearing (*e.g., via* the use of rotating blades, and/or liquid flow turbulences), through collision (e.g., by impact with beads or paddles), and/or through crushing or grinding (*e.g., via* the use of moving pistons).

In certain preferred embodiments, dilaceration is performed using a tissue dissociation or tissue homogenization machine, such as, for example, au automated tissue dissociation or homogenization instrument. Examples of automated tissue dissociation or homogenization instruments that can be used in the practice of the present invention include, but are not limited to, the GentleMACS^{®} dissociator (Miltenyi Biotec), the TissueLyser^{®} I or II systems (Qiagen), and Medi machine system (DAKO).

Preferably, when practicing the present invention in a laboratory setting, the dilaceration steps are performed using the GentleMACS^{®} dissociator. The present inventors have found that this instrument allows for an efficient but gentle dilaceration of 3D hydrogel matrices.

Preferably, in the fist dilaceration step *(i.e.,* step (a)), the dilaceration conditions are more stringent than the conditions in the second dilaceration step *(i.e.,* step (b)).

The dilaceration steps allow the recovery of more of the cells grown in the 3D hydrogels than an enzymatic step used alone.

### b - Enzymatic Degradation

Following the first dissociation step, the dilacerated hydrogel matrix is incubated with an enzyme that degrades at least one component of the 3D hydrogel matrix. It will generally be advantageous to select the enzyme based on the nature of the main component of the matrix *(i.e.,* the component that is predominantly present in the hydrogel).

In certain preferred embodiments, the enzyme specifically degrades at least one component of the 3D hydrogel matrix. Thus, for example, if the matrix is a hyaluronan hydrogel, the enzyme used will be a hyaluronidase, which specifically hydrolyzes the 1,4-linkage of hyaluronic acid; if the matrix is a collagen hydrogel, the enzyme will be a collagenase which specifically breaks peptide bonds in collagen, etc.... These "specific" enzymes have the advantage of being more selective than enzymes such as proteases that are commonly used to detach cells from 3D matrices. Indeed, these specific enzymes degrade the hydrogel without being disruptive to cell structures and functions. In contrast, proteases can digest away cell surface molecules that are essential not only for cell attachment to substrates but also for signaling molecules that are important for cell function.

Preferably, incubation of the hydrogel with the enzyme is carried out in an incubator at 37°C, under sterile conditions, and in a 5% CO₂ atmosphere. The incubation time will depend on several factors including the nature of the enzyme used, the size of the starting 3D hydrogel matrix, and the amount of enzyme present during the incubation. It is within the knowledge of one skilled in the art to determine the optimal incubation time as well as the effective amount of enzyme.

### c - Filtration and Cell Isolation

The consecutive steps of: dilaceration, enzymatic degradation and dilaceration yield a suspension of cells grown in the 3D hydrogel matrix and of fragments of 3D hydrogel matrix. In certain embodiments, a method of the present invention further comprises a step of: isolating the cells from the suspension.

Preferably, the isolation is performed *via* filtration and centrifugation under sterile conditions. More specifically, in certain embodiments, the harvesting method of the present invention further comprises steps of: (d) filtrating the suspension comprising cells grown in the 3D hydrogel matrix and fragments of the 3D hydrogel matrix using a filter; (e) washing the filter; (f) collecting the filtrate, wherein the filtrate is a suspension of cells grown in the 3D hydrogel matrix; and (g) centrifugating the filtrate to obtain a pellet consisting of cells that were grown in the 3D hydrogel matrix.

One skilled in the art will know how to select a filter that physically blocks the fragments of the hydrogel matrix but lets cells through. In embodiments where a uniform single-cell suspension is desired, it may be advantageous to use a cell strainer. Such cell strainers are commercially available, for example, from BD Biosciences, Biologix Research Company, SPL Life Sciences, VWR, and Miltenyi Biotec.

One skilled in the art will also know how to select a washing medium to wash the filter after filtration. Generally, the washing medium will be a sterile culture medium. In particular, the culture medium may be a minimal medium in which cells can survive, such as for example Dulbecco modified Eagle's minimal essential medium (DMEM) or RMPI supplemented or not with decomplemented fetal calf serum (FCS).

Similarly, one skilled in the art will know how to select centrifugation conditions to obtain a cell pellet from a cell suspension. The cells obtained are healthy, viable and fully functioning; and therefore may be used immediately following this isolation step.

### 2 - Suitable 3D Cell Cultures

A harvesting method according to the invention may be carried out on any of a variety of 3D cell cultures in hydrogels.

### a - Suitable 3D Hydrogel Matrices

Suitable 3D hydrogel matrices include any hydrogel that has been designed for 3D cell culture or is suitable for 3D cell culture. The hydrogel may consist of a single macromolecule component or, alternatively, it may comprise at least two macromolecule components. The components of a hydrogel may be naturally-occurring macromolecules, modified biopolymers or synthetic polymers.

Examples of naturally-occurring macromolecules that have been used to prepare hydrogels for 3D cell culture include, but are not limited to, collagen, gelatin, hyaluronic acid, fibrin, alginate, agarose, cellulose, Matrigel^{®}, and chitosan. Examples of modified biopolymers that have been used to prepare hydrogels for 3D cell culture include, but are not limited to, derivatized celluloses such as methylcelluloses, derivatized hyaluronic acid such as amine-modified hyaluronic acids and esterified hyaluronic acids, derivatized collagens such as amino-modified collagens and esterified collagens. Examples of synthetic polymers that have been used to prepare hydrogels for 3D cell culture include, but are not limited to, poly(acrylic acid) and its derivatives, poly(ethylene oxide) and its copolymers, poly(vinyl alcohol), poly(ethylene glycol), poly(2-hydroxy-ethyl methacrylate, polyphosphazene and polycaprolactone.

Hydrogels suitable for use in a method of the present invention may be prepared by any suitable method. Such methods are known in the art. Alternatively, the hydrogels may be purchased from commercial sources. Examples of commercially available 3D hydrogel matrices include, but are not limited to, MaxGel^{®} human Extracellular Matrix (ECM), HydroMatrix^{®} human ECM, and Mouse ECM which can be purchased from Sigma; Extracel^{®} which is manufactured by Glycosan Biosystems, and Geltrex^{®} matrix proposed by Invitrogen.

Suitable 3D hydrogel matrices may further comprise any of a variety of biomolecules, whose presence is desirable in a hydrogel intended to be used as a scaffold for 3D cell culture. Examples of such biomolecules include, but are not limited to, proteoglycans or glycosaminoglycan chains (such as heparin chondroitin sulfate, dermatan, heparin sulfate or proteoglycans thereof), hormones (such as insulin, transferrin, growth hormone, tri-iodothyronine, glucagon, and the like), growth factors (such as epidermal growth factor (EGF), fibroblast growth factor (FGF), transforming growth factor (TGF-β), hepatocyte growth factor (HGF), leukemia inhibitory factor (LIF), interleukin 6 (IL-6), interleukin 11 (IL-11), interleukin 13 (IL-13), interleukin-8 (IL-8), interleukin 3 (IL-3), interleukin 5 (IL-5), granulocyte macrophage stem cell factor (GM-SCF), granulocyte stem cell factor (G-SCF), erythropoietin (EPO), thrombopoietin (TPO), oncostatin M); chemoattractants (such as peptides integrin motif (RGD), collagen, Kelastin derived motif (VGVAPG)₃ and chemokines SDF1-α) and the like.

Suitable 3D hydrogel matrices may comprise a macromolecule crosslinked with another molecule, such as, for example, an integrin motif (RGD), Kelastin, or a peptide fragment (VGVAPG)₃. Alternatively or additionally, suitable 3D hydrogel matrices may be coated with another molecule, such as, for example, poly-L-lysine, RGD motif, and the like.

Alternatively or additionally, a 3D hydrogel matrix may further comprise at least one of a variety of biologically active agents, pharmaceutically active agents, and any combinations thereof.

Methods for preparing hydrogels comprising, or coated with, biomolecules, biologically active agents or pharmaceutically active agents are known in the art. Alternatively, the biomolecules, biologically active agents and/or pharmaceutically active agents may be added to a 3D hydrogel matrix by pre-incubating the hydrogel with a cell culture medium comprising such a biomolecule, biologically active agent or pharmaceutically active agent prior to seeding cells.

In certain preferred embodiments, prior to harvest, the cell culture is carried out in a cross-linked hyaluronan hydrogel. Hyaluronan is (with collagen) the principal constituent of the extracellular matrix (ECM). Hyaluronan is a glycosaminoglycan composed of a repeating disaccharide of glucuronic acid and N-acetylglycosamine (β1,4-GlcUA-β1,3-GlcNAc)ₙ. It contributes significantly to cell proliferation and migration, and participates in a number of cell surface receptor interactions. It is generally accepted that hyaluronan is also implicated in tumor progression (Stern, Pathol. Biol., 2005, 53: 372-382). Hyaluronans are glycosaminoglycans (GAGs) consisting of disaccharide units that are themselves composed of D-glucuronic acid and D-N-acetylglucosamine, linked together *via* alternating β-1,4 and β-1,3 glycosidic bonds. Therefore, considerable efforts have focused on the use of hyaluronan as a material for 3D hydrogel matrices.

Examples of cross-linked hyaluronan hydrogels include, but are not limited to, the hydrogels described in Prestwich et al., J. Control. Release, 1998, 53: 93-103; Rowley et al., Biomaterials, 1999, 20: 45-53; Comisar et al., Biomaterials, 2007, 28: 4409-4417; Ferreira et al., Biomaterials, 2007, 28: 2706-2717; and Chua et al., Biomaterials, 2008, 29: 1412-1421; the reticulated hyaluronan hydrogel suitable developed in the laboratory of the present inventors and used to examine cancer cell invasion in 3D and evaluate cancer cell sensitivity to anticancer drugs (David et al., Matrix Biology, 2004, 23: 183-193; David et al., Cell Prolif., 2008, 41: 348-364; David et al., Acta Biomaterialia, 2008, 4: 256-263; Coquerel et al., Glia, 2009, 57: 1716-1726); and hyaluronan hydrogels commercially available from Glycosan Biosystems (Extracel^{®}), Lifecore Biomedical LLC, and BD Biosciences (BD Matrigel™ Basement Membrane Matrix).

However, preferably, prior to cell culture, a crosslinked hyaluronan hydrogel is processed or treated according to a method developed in the laboratory of the present inventors. This method is described in a European patent application number EP 10 305 666 and European patent application number EP 11 305 333 (each of which is incorporated herein by reference in its entirety). Briefly, preferably, prior to cell culture, a crosslinked hyaluronan hydrogel is: (i) lyophilized; and (ii) sterilized first by heating the hydrogel for 30 minutes to 1 hour using an oil bath at 100°C, and then by immersing the hydrogel in pure ethanol at room temperature and submitting the immersed hydrogel to physical compression.

### 3 - Suitable Cell Cultures

Cells that can be harvested according to a method of the present invention include any cell that can be grown on a 3D hydrogel matrix. These include stem cells, induced pluripotent stem cells, progenitor cells, and differentiated cells. The harvesting method described herein is particularly advantageous in the case of stem cells and progenitor cells which are known to be more fragile in terms of manipulation than adult differentiated cells.

Cells to be harvested using a method of the present invention may be of a single cell types (*e.g.,* cardiomyocytes or fibroblasts) or may comprise at least two different cell types (e.g., keratinocyte-fibroblast co-culture). Preferably, cells to be harvested according to the present invention are of mammalian (animal or human) origin. Mammalian cells may be of any organ, fluid or tissue origin (*e.g.,* brain, liver, skin, lung, kidney, heart, muscle, bone, bone marrow, blood, amniotic fluid, umbilical cord blood, etc) and of any cell type (see below). Cells may be primary cells, secondary cells or immortalized cells *(i.e.,* established cell lines). They may be isolated or derived from *ex vivo* biological samples or obtained from volunteers or patients by techniques well known in the art, or alternatively they may be purchased from commercial resources (for example, from the American Type Culture Collection, Manassas, VA). Alternatively or additionally, cells may be genetically engineered to contain a gene of interest such as a gene expressing a growth factor or a receptor, or to contain a defective gene, or yet to contain Oct3/4, Sox2, Klf4, and c-Myc genes in order to prepare human induced stem cells from adult somatic cells.

Examples of adult differentiated cells that can be grown in a 3D hydrogel matrix include, but are not limited to, basal cells, epithelial cells, platelets, lymphocytes, T-cells, B-cells, natural killer cells, reticulocytes, granulocytes, monocytes, mast cells, neurocytes, neuroblasts, glioblastom, cytomegalic cells, dendritic cells, macrophages, blastomeres, endothelial cells, interstitial cells, Kupffer cells, Langerhans cells, littoral cells, tissue cells such as muscle cells and adipose cells, osteoblasts, fibroblasts, and the like.

Examples of progenitor cells that can be grown in a 3D hydrogel matrix include, but are not limited to, hematopoietic progenitor cells, endothelial progenitor cells, neural progenitor cells, mesenchymal progenitor cells, osteogenic progenitor cells, stromal progenitor cells, and the like. The present inventors have demonstrated the feasibility of 3D culture of hematopoietic progenitor cells in a crosslinked hyaluronan hydrogel and the possibility of harvesting such cells using a method of the present invention (see Examples section).

Examples of stem cells that can be grown in a 3D hydrogel matrix include, but are not limited to, embryonic stem cells, adult stem cells and induced pluripotent stem cells.

Prior to harvesting, the cell culture is conducted using "***appropriate culture medium and conditions".*** The term "appropriate culture medium and conditions" refers to a culture medium and to conditions that support or favor survival and proliferation of cells cultured in a matrix. Such culture media and conditions are known in the art or may easily be optimized by one skilled in the art. The Examples section below show a comparison of different conditions for the culture of hematopoietic progenitor cells in a crosslinked hyaluronan hydrogel.

### II - Isolated Cells

Cells harvested by a method described herein (*i.e*., isolated cells) are also disclosed. As used herein, the term "***isolated cells**"* refers to cells that have been detached or dissociated from the 3D hydrogel matrix in which they have been cultured. Thus, this term encompasses cells that are in suspension together with hydrogel fragments resulting from dilaceration and enzymatic degradation, as well as cells that have been separated from these hydrogel fragments by filtration.

The cells disclosed herein may be of a single type (*i.e.,* constitute a homogeneous population) or may comprise at least two different cell types (*i.e.,* constitute a heterogeneous population), as described above.

The cells dislosed herein are healthy, viable and fully functioning and can be used immediately after being harvested.

### III - Uses of Harvested Cells

The harvesting method described herein renders cells grown on 3D hydrogel matrices more readily available and consequently more easily and widely usable. Indeed, the efficiency and non-disruptive effects of the harvesting method allows for large-scale production of functional cells, including stem cells and progenitor cells, which can be used in a large variety of applications.

Thus, for instance, cells harvested according to a method of the present invention may be used in cell implantation applications such as in the treatment of human clinical conditions (*e.g.,* cancer, infectious diseases) or in regenerative medicine (*e.g.,* for the restoration and repair of damaged, injured or defective tissues). The availability of large quantities of functioning stem cells, which have been proposed as promising candidates for future therapies, could facilitate the development of autologous (from the patient) or allogeneic (from another donor) transplantations. Similarly, using 3D hydrogel matrices and a harvesting method of the present invention, large quantities of cells from genetically-customized stem cell lines could be produced for cell-based therapy. The combination of a 3D cell culture and a harvesting method described herein could also be used for the proliferation of stem cells in therapeutic cloning (also called somatic cell nuclear transfer).

Alternatively, cells harvested according to a method of the present invention may be used in tissue engineering applications. Indeed, since the cells obtained are fully functional and do not require to recover from the typical detachment damages experienced when standard harvesting methods are used, they can be immediately seeded in a scaffold and can efficiently interact with the scaffold surfaces.

The availability of large quantities of functional cells is also an advantage in cell biology research.

### IV - Kits

In another aspect, kits are provided that comprise: (a) a crosslinked hyaluronan hydrogel processed or treated according to a method developed in the laboratory of the present inventors and described in European patent application number EP 10 305 666 and European patent application number EP 11 305 333, and (b) an amount of hyaluronidase sufficient to harvest cells according to a method of the present invention.

The crosslinked hyaluronan hydrogel comprised in the kit may be a sterilized crosslinked hyaluronan hydrogel contained in a container under sterile conditions, or a rehydrated crosslinked hyaluronan hydrogel comprised in a rehydrating medium comprising antibiotics.

A kit of the invention may further comprise instructions for storing the sterilized hydrogel, and/or instructions for rehydrating the sterilized hydrogel, instructions for storing the rehydrated hydrogel, and/or instructions for using the rehydrated hydrogel. A kit may further comprise one or more of: rehydrating medium and/or reagents; antibiotics; biomolecules, biologically active agents, and/or pharmaceutically active agents as described herein, cell culture medium and/or reagents, cells, seeding means, harvesting medium and/or reagents, filters, washing medium and/or reagents, and the like.

The different additional reagents included in an inventive kit may be supplied in a solid (*e.g.,* lyophilized) or liquid form. The kits of the present invention may optionally comprise different containers (*e.g.,* vial, ampoule, test tube, flask or bottle) for each individual buffer and/or reagent. Each component will generally be suitable as aliquoted in its respective container or provided in a concentrated form. Other containers (*e.g.,* vials, ampoules, test tubes, flasks, bottles or assay plates) suitable for conducting certain procedures (*e.g.,* rehydration, cell culture, dilaceration, incubation, filtration, etc...) may also be provided. The individual containers of the kit are preferably maintained in close confinement for commercial sale.

An identifier, *e.g.,* a bar code, radio frequency, ID tags, etc., may be present in or on the kit. The identifier can be used, for example, to uniquely identify the kit for purposes of quality control, inventory control, tracking movement between workstations, etc.

### Examples

The following examples describe some of the preferred modes of making and practicing the present invention. However, it should be understood that the examples are for illustrative purposes only and are not meant to limit the scope of the invention. Furthermore, unless the description in an Example is presented in the past tense, the text, like the rest of the specification, is not intended to suggest that experiments were actually performed or data were actually obtained.

### Example 1: Synthesis of Crosslinked Hyaluronan Hydrogels

The hyaluronan (HA) hydrogels used were prepared using a long-chain of hyaluronan cross-linked with adipic dihydrazide (ADH, Sigma) as cross-linking agent and 1-ethyl-3[3-(dimethylamino)-propyl]carbodiimide (EDCI, Sigma) as reagent. All the hydrogels were prepared from high molecular weight hyaluronan (> 1x10⁶ Da) according to the procedure described by Prestwich et al. (J. Control. Release, 1998, 53: 93-103). Briefly, the ratios of ADH to hyaluronan and of hyaluronan to EDCI were adjusted to obtain hydrogels optimized for cell adhesion and culture. The best results were obtained with a ratio of ADH to hyaluronan equal to 10:1 and a ratio of hyaluronan to EDCI equal to 1:1. Hyaluronan and hydrazide cross-linker (ADH) were dissolved in milliQ-water and the pH was adjusted to 4 by adding 0.1 N HCl. The carbodiimide reagent (EDCI) was dissolved in milliQ-water and added to the reaction mixture, and allowed to gel for 2 hours with gentle agitation. Hyaluronan hydrogels were equilibrated in 0.1 N NaCl for 2 days, then in a mixture water/ethanol (3/1, v/v) for 2 days, and in milliQ-water for 2 days to remove ADH.

Crosslinked hyaluronan hydrogels thus obtained were lyophilized. Each hydrogel was dialyzed, placed in a plastic container and frozen. Following freezing, the hydrogels were placed in a lyophilizer (Alpha 1-2 - performances: 2 kg of ice per 24 hours, T=-55°C). Depending on the volume of water to be eliminated, the lyophilisation was carried out for 4 to 5 days. The lyophilized hydrogels were then stored at -20°C.

Prior to use, the lyophilized hydrogels were cut into rectangular parallelepipeds (about 5 x 5 x 1.5 mm). The cut hydrogels were then sterilized at 100°C for 1 hour using an oil bath. The hydrogels were then immersed in pure ethanol and manually compressed to eliminate air trapped within the hydrogel structure. The hydrogels were then rehydrated using a cell culture medium (*e.g.,* RPMI (Eurobio) comprising antibiotics) until they swelled by a 100-fold in volume.

Some hydrogels were coated with the tripeptide RGD motif containing in Pronectin F Plus® (Sigma) at three different concentrations (50 ng/mL, 100 ng/mL, 150 ng/mL).

Prior to cell seeding, each hydrogel was incubated for 24 hours with RPMI enriched in human stromal cell-derived factor 1α (SDF-1α, R&D System) (100 ng/mL). The presence of SDF-1α, which is a chemokine (*i.e.,* a cytokine with a chemotactic effect), not only attracts the hematopoietic progenitor cells to the hydrogel but will also stimulates the secretion of proteolytic enzymes which promote circulation and proliferation of cells in the hydrogel.

### Example 2: 3D Culture of Hematopoietic Progenitor Cells

***Isolation of Human Hematopoietic Progenitor Cells.*** Mononuclear hematopoietic cells from peripheral blood (PBMC) were obtained from healthy and consentant volunteers. The PBMCs were isolated by gradient density separation (d=1.077, Percoll, Sigma). The cells obtained were then washed twice in Hank's medium (Eurobio), and then submitted to a depletion step to only collect non-adherent mononuclear cells. These non-adherent mononuclear cells comprise hematopoietic progenitor cells, including erythrocyte progenitor cells, granulocyte progenitor cells, and megakaryocyte progenitor cells.

In another series of experiments, the cells isolated by gradient density separation were submitted to a magnetic selection in order to collect CD34⁺ hematopoietic stem cells. This selection was then followed by flow cytometry analysis to determine the percentage of CD34⁺ hematopoietic stem cells present in the cell suspension.

***3D Culture of Human Hematopoietic Progenitor Cells.*** In order to optimize the number of cells to be retrieved, each experiment was carried out using 3 hydrogels for each experimental condition. Three crosslinked hyaluronan hydrogels prepared as described above were placed in a culture well together with 3 x 10⁶ or 9 x 10⁶ human hematopoietic progenitor cells or with 2.5x10⁴ CD34⁺ hematopoietic stem cells comprised in 2 mL of Stemspan (StemCell) containing 50 ng/mL Stem Cell Factor (SCF, R&D Systems) and 0.1% antibiotics. The hydrogels and cells were left to stand at 37°C and in a 5% CO₂ atmosphere for 24 hours, during which time the cells migrated into and colonized the hydrogels. The hydrogels were then placed in different culture wells containing Stemspan in the presence of SCF (50 ng/mL), VEGF (10 ng/mL) (R&D Systems), and angiopoietin-1 (Ang-1) (300 ng/mL) (R&D Systems), or with hydrogels coated or not (see Figure 4). The hydrogels were then incubated for 7 days, 14 days, 28 days, 42 days or 56 days. Each week, half of the culture medium was removed and replaced with new culture medium.

### Example 3: Retrieval of Hematopoietic Progenitor Cells Grown in 3D

Three hydrogels cultured in a same well were removed from the culture well and placed in a C-Tube (Miltenyi Biotec). The culture medium left in the culture well was collected and cells present in the culture medium (*i.e.,* cells that had migrated out of the hydrogels) were isolated.

To each C-tube containing three hydrogels was added 3 mL of medium (RPMI + 10% fetal calf serum (FCS, Eurobio)). Each C-tube was then placed in a Gentle Macs^{®} (Miltenyi Biotec), and the content of each tube was submitted to a dilaceration according to a pre-recorded program called m_brain_01 (initially developed for the preparation of single cell suspensions from mouse neural tissue and which lasts for 37 seconds). Hyaluronidase (H4272, Sigma) was then added to each C-tube at a concentration of 150 UI/mL and each tube was placed in an incubator (37°C and 5% CO₂) for 45 minutes. Each C-tube was then placed in the Gentle Macs^{®} and the content of each tube was submitted to a dilaceration according to a pre-recorded program called m_brain_02 (initially developed for the preparation of single cell suspensions from mouse neural tissue and which lasts for 31 seconds). This yielded a suspension.

The suspension was filtered using a pre-separation filter (Miltenyi Biotec) placed on a 15 mL-tube, and the filter was washed with 10 mL of RPMI. The filtrate obtained *(i.e.,* the cell suspension) was centrifuged at 1500 rpm for 10 minutes and the cells were collected.

Thus, for each of the following incubation times: D0, D7, D14, D28, D42 and D56, the present inventors have separately retrieved (1) hematopoietic progenitor cells that were grown in three hyaluronan hydrogels and that had stayed inside these hydrogels and (2) hematopoietic progenitor cells that were grown in the same three hyaluronan hydrogels but that had migrated out of these hydrogels.

### Example 4: Characterization of Hematopoietic Progenitor Cells Grown in 3D

The cells of each cell sample obtained in Example 3 were resuspended in a semi-solid medium (Stemα4B, STEMα) comprising IMDM, SVF, human transferrin, L-glutamin, 1-monothioglycerol, cytokines (IL-3, IL-6, IL-11, SCF, EPo, GM-SCF, G-CSF, Flt-3L) and human purified collagen solution, and cultured for 14 days. The colonies formed were evaluated *(i.e.,* colony forming unit - CFU - was determined) and the colony cells were morphologically identified allowing a distinction between the erythroblastic colonies (BFU-E), macrophagic and granulomonocytic colonies (CFU-GM) and mixed colonies (CFU-GEMM).

In addition, the cells of each cell sample obtained in Example 3 were further identified *via* May-Griinwald-Giemsa (MGG) staining.

### Results Obtained

***Evaluation of Different Culture Conditions.*** The histogram presented in Figure 1 shows the mean CFU values determined for each set of three hydrogels, as a function of culture time, for 4 different experimental conditions: 50 ng/mL SCF (condition 1), 50 ng/mL SCF + 10 ng/mL of VEGF (condition 2), 50 ng/mL SCF + 300 ng/mL Ang-1 (condition 3), and 50 ng/mL SCF + 10 ng/mL of VEGF + 300 ng/mL Ang-1 (condition 4). The mean values are expressed in CFU/mm³ of hydrogel.

In the case of conditions 1 and 2, a sharp decrease in (almost a disappearance of) the number of all types of hematopoietic progenitor cells was observed from D14. In the case of condition 3, a decrease in the total number of progenitor cells was also observed at D14, however, at D14 and after, hematopoietic cells of only one type were present: the CFU-GM cells. In the case of condition 4, the total number of progenitor cells at D14 appeared to be higher than at any other prior time; and the cell population comprised 2 types of hematopoietic progenitor cells, BFU-E and CFU-GM cells. These two types of cells were present up to at least D28.

When the hematopoietic cells that had stayed in the hydrogels were analyzed microscopically, no differences were observed (see Figure 2) as a function of culture time. However, in the case of condition 4, at D56, the presence of two types of cell populations was observed: a population of small cells and a population of large cells.

The hematopoietic cells that had migrated inside the hydrogels and that had been collected at different culture times were also analyzed microscopically (Figure 3). At D0, no significant morphological differences were observed. However at D28, in the case of condition 4, two types of cells were observed (small cells and large cells) while in the case of condition 1, cells of a single type were present (large cells). Under these 2 conditions (condition 1 and condition 4), the volume of the small cells was found to increase as the culture time increased, which could suggest cell differentiation.

The results obtained showed that the combination SCF, VEGF and Ang-1 *(i.e.,* condition 4) allowed the simultaneous survival of 2 types of hematopoietic progenitor cells: the CFU-GM and BFU-E cells. Starting D14, the combination SCF and Ang-1 *(i.e.,* condition 3) allowed the survival of one type of hematopoietic progenitor cells: the CFU-GM cells.

***Evaluation of the Effect of Coating of Hyaluronan With a Tripeptide RGD**.* The results obtained are presented on Figure 4 where 2D represents culture without hydrogel, 3D represents culture with hydrogel non-coated, and 3D RGD represents culture with hydrogel coated in the presence of 50 ng/m of RGD. In all these conditions, the cell cultures were carried out using a medium comprising SCF (50 ng/mL) + 0.1% antibiotics and the cell harvesting methods used as described above.

At D28, the number of cells in the 3D culture and the number of cells in the 3D RGD culture were each higher (p<0.01) than the number of cells in the 2D culture. Furthermore, at 28 days of culture, a higher survival of progenitors and a higher number of progenitors harvested were observed in the case of hydrogels coated with RGD.

### Conclusions

The results obtained clearly show that crosslinked hyaluronan hydrogels can be used for the long term culture of hematopoietic progenitor cells, and therefore are suitable for use in the investigation of early stages of hematopoiesis. Indeed, these hydrogels have proved to be non-toxic media that allow survival of hematopoietic progenitor cells. In the present experiments, survival of hematopoietic progenitor cells inside the hydrogels was observed at D28 using a culture medium comprising a simple combination of cytokines (SCF, VEGF and Ang-1). Coating of hyaluronan with the tripeptide RGD was found to promote the entry of hematopoietic progenitor cells in a dose-dependent manner until D28. This result suggests that co-crosslinking prevents a stable binding between the progenitor cells and the RGD motif.

In addition, the results obtained have shown that the method for retrieving progenitor cells grown in a 3D hydrogel matrix is simple and efficient. Indeed, using this method, the present inventors have been able to establish that progenitor cell colonies cultured for 28 days in hydrogels in the presence of SCF, VEGF and Ang-1 were mainly of two types: macrophagic and granulomonocytic progenitors (CFU-GM) and erythroblastic progenitors (BFU-E). In contract, in the presence of SCF and VEGF, the only progenitor cells surviving after 28 days of culture in hydrogels are CFU-GMs.

The results presented above demonstrate the value of the harvesting method of the present invention.

### Example 5: Viability of Hematopoietic Progenitor Cells Grown in 3D

The viability of hematopoietic progenitor cells cultured in 3D crosslinked hyaluronan hydrogels as described in Examples 1 and 2 and harvested as described in Example 3 was analyzed. Viability was measured using an automatic viability cell counter named ADAM (Labtech, France). This counting device is based on the combined use of laser fluorescence microscopy and a camera. Two aliquots are prepared from each cell sample. In a first step, cells of one aliquot are stained using propidium iodide and automatically counted (this measurement provides the number of dead cells in the aliquot). In a second step, cells of the second aliquot are stained using propidium iodide and contacted with a detergent and then automatically counted (this measurement provides the number of dead cells and live cells in the aliquot).

The viability was measured 28 days and 56 days after seeding of the cells. Four different culture conditions were used: 3D matrix in the presence of 50 ng/mL SCF (condition A); 3D matrix in the presence of 50 ng/mL SCF + 50 ng/mL PTN (condition B); 3D matrix coated with tripeptide RGD in the presence of 50 ng/mL SCF; (condition C); and 3D matrix coated with tripeptide RGD in the presence of 50 ng/mL SCF + 50 ng/mL PTN (condition D). The results obtained are presented in the following table.

**Table 1. Viability of hematopoietic progenitor cells (n=4) cultured in 3D under different conditions and harvested according to the method of the present invention.**

| | | Condition A | | Condition B | | Condition C | | Condition B | |
|---|---|---|---|---|---|---|---|---|---|
| | | 3D and SCF | | 3D and SCF + PTN | | 3D RGD and SCF | | 3D RGD and SCF + PTN | |
| Mean viability | Day 28 | 96% | 66% | 95% | 73% | 96% | 62% | 95% | 51% |
| | Day 56 | 97% | 51% | 95% | 55% | 96% | 52% | 96% | 67% |

The results obtained show that the viability of hematopoietic progenitor cells cultured in crosslinked hyaluronan hydrogels and harvested using a method of the present invention was almost identical at day 28 and day 56. Indeed, the viability was found to be between 51 % and 96% (with a mean value of 79%) after 28 days of culture and between 51% and 97% (with a mean value of 76%) after 56 days of culture. The culture conditions were not found to significantly affect the viability of the cells.

### Example 6: Morphology of Hematopoietic Progenitor Cells Grown in 3D

A semi-quantitative analysis of the different types of cells present in the hydrogels (prepared and cultured as described in Examples 1 and 2) and present in the supernatant of these hydrogels (*i.e.,* cells that had migrated out of the hydrogels) was performed by optical microscopy after May-Griinwald-Giemsa (MGG) staining. This analysis allowed a distinction between erythroid precursors, granulocyte precursors, megakaryocyte precursors, and monocytes/macrophages.

Four different culture conditions were used: conditions A, B, C and D, as described in Example 4. The results obtained 1 day after cell seeding and 28 days after cell seeding are presented in the following tables.

**Table 2. Semi-quantitative analyse of the cells present in the supernatant (S) or in the hydrogel (H) under different culture conditions at Day 1 and Day 28after seeding.**

| | Condition A | | Condition B | | Condition C | | Condition B | |
|---|---|---|---|---|---|---|---|---|
| | 3D and SCF | | 3D and SCF + PTN | | 3D RGD and SCF | | 3D RGD and SCF + PTN | |
| **Day 1** | S | H | S | H | S | H | S | H |
| erythroid | +++ | + | +++ | - | ++ | ++ | +++ | - |
| granulocyte | + | + | - | - | - | - | + | - |
| megakaryocyte | - | - | - | - | - | - | - | - |
| monocytes macrophages | + | - | + | - | - | - | + | - |

| **Day 28** | | | | | | | | |
|---|---|---|---|---|---|---|---|---|
| erythroid | ++ | + | ++ | + | + | ++ | + | +++ |
| granulocyte | - | - | - | - | - | - | - | - |
| megakaryocyte | - | - | - | - | - | - | - | - |
| monocytes macrophages | +++ | +++ | +++ | +++ | +++ | +++ | +++ | +++ |

| | | | | | | | | |
|---|---|---|---|---|---|---|---|---|
| +++ = high content, ++ = medium content, + = low content, - = no cells. | | | | | | | | |

The results presented above show some homogeneity between the different types of cells in the different culture conditions. After 28 days of culture, proliferation is observed in all the culture conditions, with a preponderance of monocytes and macrophages. At that time, erythroid precursors are still present in all the conditions, but more so in condition D (hydrogels coated with RGD in the presence of PTN).

It is intended that the specification and examples be considered as exemplary only, with the true scope of the invention being indicated by the following claims.

## Claims

1. A method for retrieving cells grown in a 3D hydrogel matrix, said method comprising steps of:
(a) submitting the 3D hydrogel matrix containing cells to a dilaceration to obtain a dilacerated hydrogel matrix;
(b) incubating the dilacerated hydrogel matrix with an enzyme that specifically degrades at least one component of the 3D hydrogel matrix to obtain an enzymatically degraded hydrogel matrix; and
(c) submitting the enzymatically degraded hydrogel matrix to a dilaceration to obtain a suspension comprising cells grown in the 3D hydrogel matrix and fragments of the 3D hydrogel matrix.

2. A method according to claim 1, wherein the step of incubating the dilacerated hydrogel matrix with an enzyme is performed in an incubator at 37°C, in a 5% CO₂ atmosphere, under sterile conditions.

3. A method according to claim 1 or claim 2, wherein the enzyme specifically degrades at least one component of the 3D hydrogel matrix.

4. A method according to any one of claims 1-3, wherein the dilacerations of steps (a) and (c) are performed using a tissue dissociation or homogenization instrument.

5. A method according to claim 4, wherein the tissue dissociation or homogenization instrument is automated.

6. A method according to any one of claims 1-5, wherein the dilacerations of steps (a) and (c) are performed under sterile conditions.

7. A method according to any one of claims 1-6, further comprising steps of:
(d) filtrating the suspension comprising cells grown on the 3D hydrogel matrix and fragments of the 3D hydrogel matrix using a filter;
(e) washing the filter; and
(f) collecting the filtrate, wherein the filtrate is a suspension of cells grown on the 3D hydrogel matrix.

8. A method according to claim 7, further comprising a step of:
(g) centrifugating the filtrate to obtain cells that were grown on the 3D hydrogel matrix.

9. A method according to any one of claims 1-8, wherein the cells grown on the 3D hydrogel matrix are of a single cell type.

10. A method according to any one of claims 1-8, wherein the cells grown on the 3D hydrogel matrix comprise at least two types of cells.

11. A method according to any one of claims 1-10, wherein the cells grown on the 3D hydrogel matrix belong to the group consisting of stem cells, induced pluripotent stem cells, progenitor cells, differentiated cells, and combinations thereof.

12. A method according to any one of claims 1-11, wherein the 3D hydrogel matrix is a 3D crosslinked hyaluronan hydrogel and the enzyme used in step (b) is hyaluronidase.

13. A method according to claim 12, wherein, prior to cell culture, the 3D crosslinked hyaluronan hydrogel is:
(i) lyophilized; and
(ii) sterilized
first by heating the hydrogel, and
then by immersing the hydrogel in pure alcohol and submitting the immersed hydrogel to physical compression.

14. A method according to any one of claims 1-13, wherein the cells harvested are healthy, viable, and fully functioning.

## Patentansprüche

1. Verfahren zum Gewinnen von in einer 3D-Hydrogel-Matrix gewachsenen Zellen, wobei das Verfahren die Schritte umfasst:
(a) Zerteilen der 3D-Hydrogel-Matrix, die die Zellen enthält, um eine zerteilte Hydrogel-Matrix zu erhalten;
(b) Inkubieren der zerteilten Hydrogel-Matrix mit einem Enzym, das spezifisch mindestens eine Komponente der 3D-Hydrogel-Matrix abbaut, um eine enzymatisch abgebaute Hydrogel-Matrix zu erhalten; und
(c) Zerteilen der enzymatisch abgebauten Hydrogel-Matrix, um eine Suspension zu erhalten, welche die in der 3D-Hydrogel-Matrix gewachsenen Zellen und Fragmente der 3D-Hydrogel-Matrix enthält.

2. Verfahren nach Anspruch 1, wobei der Schritt der Inkubation der zerteilten Hydrogel-Matrix mit einem Enzym in einem Inkubator bei 37 °C in 5 % CO₂-Atmosphäre unter sterilen Bedingungen durchgeführt wird.

3. Verfahren nach Anspruch 1 oder Anspruch 2, wobei das Enzym spezifisch mindestens eine Komponente der 3D-Hydrogel-Matrix abbaut.

4. Verfahren nach einem der Ansprüche 1-3, wobei die Zerteilungen der Schritte (a) und (c) unter Verwendung eines Gewebeauflösungs- oder Homogenisierungsgeräts durchgeführt werden.

5. Verfahren nach Anspruch 4, wobei das Gewebeauflösungs- oder Homogenisierungsgerät automatisiert ist.

6. Verfahren nach einem der Ansprüche 1-5, wobei die Zerteilungen der Schritte (a) und (c) unter sterilen Bedingungen durchgeführt werden.

7. Verfahren nach einem der Ansprüche 1-6, ferner umfassend die Schritte:
(d) Filtrieren der Suspension, welche auf der 3D-Hydrogel-Matrix gewachsene Zellen und Fragmente der 3D-Hydrogel-Matrix enthält, unter Verwendung eines Filters;
(e) Waschen des Filters; und
(f) Sammeln des Filtrats, wobei das Filtrat eine Suspension von auf der 3D-Hydrogel-Matrix gewachsenen Zellen ist.

8. Verfahren nach Anspruch 7, ferner umfassend einen Schritt von:
(g) Zentrifugieren des Filtrats, um Zellen zu erhalten, die auf der 3D-Hydrogel-Matrix wachsen gelassen wurden.

9. Verfahren nach einem der Ansprüche 1-8, wobei die auf der 3D-Hydrogel-Matrix gewachsenen Zellen aus einem einzigen Zelltyp sind.

10. Verfahren nach einem der Ansprüche 1-8, wobei die auf der 3D-Hydrogel-Matrix gewachsenen Zellen mindestens zwei Typen von Zellen umfassen.

11. Verfahren nach einem der Ansprüche 1-10, wobei die auf der 3D-Hydrogel-Matrix gewachsenen Zellen zu der Gruppe, bestehend aus Stammzellen, induzierten pluripotenten Stammzellen, Vorläuferzellen, differenzierten Zellen und Kombinationen davon, gehören.

12. Verfahren nach einem der Ansprüche 1-11, wobei die 3D-Hydrogel-Matrix ein 3D-vernetztes Hyaluronan-Hydrogel ist und das in Schritt (b) verwendete Enzym Hyaluronidase ist.

13. Verfahren nach Anspruch 12, wobei das 3D-vernetzte Hyaluronan-Hydrogel vor der Zellkultur:
(i) lyophilisiert und
(ii) sterilisiert,
zuerst durch Erhitzen des Hydrogels und
dann durch Eintauchen des Hydrogels in reinen Alkohol und physikalisches Komprimieren des eingetauchten Hydrogels,
wird.

14. Verfahren nach einem der Ansprüche 1-13, wobei die geernteten Zellen gesund, lebensfähig und voll funktionsfähig sind.

## Revendications

1. Procédé de récupération de cellules cultivées dans une matrice d'hydrogel en 3D, ledit procédé comprenant les étapes de :
(a) soumission de la matrice d'hydrogel en 3D contenant des cellules à une dilacération pour obtenir une matrice d'hydrogel dilacérée ;
(b) incubation de la matrice d'hydrogel dilacérée avec une enzyme qui dégrade spécifiquement au moins un composant de la matrice d'hydrogel en 3D pour obtenir une matrice d'hydrogel enzymatiquement dégradée ; et
(c) soumission de la matrice d'hydrogel enzymatiquement dégradée à une dilacération pour obtenir une suspension comprenant des cellules cultivées dans la matrice d'hydrogel en 3D et des fragments de la matrice d'hydrogel en 3D.

2. Procédé selon la revendication 1, dans lequel l'étape d'incubation de la matrice d'hydrogel dilacérée avec une enzyme est réalisée dans un incubateur à 37 °C, dans une atmosphère contenant 5% de CO₂, dans des conditions stériles.

3. Procédé selon la revendication 1 ou la revendication 2, dans lequel l'enzyme dégrade spécifiquement au moins un composant de la matrice d'hydrogel en 3D.

4. Procédé selon l'une quelconque des revendications 1 à 3, dans lequel les dilacérations des étapes (a) et (c) sont réalisées en utilisant un instrument de dissociation ou d'homogénéisation de tissus.

5. Procédé selon la revendication 4, dans lequel l'instrument de dissociation ou d'homogénéisation de tissus est automatique.

6. Procédé selon l'une quelconque des revendications 1 à 5, dans lequel les dilacérations des étapes (a) et (c) sont réalisées dans des conditions stériles.

7. Procédé selon l'une quelconque des revendications 1 à 6, comprenant en outre les étapes de :
(d) filtration de la suspension comprenant les cellules cultivées sur la matrice d'hydrogel en 3D et des fragments de la matrice d'hydrogel en 3D en utilisant un filtre ;
(e) lavage du filtre ; et
(f) recueil du filtrat, où le filtrat est une suspension de cellules cultivées sur la matrice d'hydrogel en 3D.

8. Procédé selon la revendication 7, comprenant en outre une étape de :
(g) centrifugation du filtrat pour obtenir des cellules qui ont été cultivées sur la matrice d'hydrogel en 3D.

9. Procédé selon l'une quelconque des revendications 1 à 8, dans lequel les cellules cultivées sur la matrice d'hydrogel en 3D appartiennent à un seul type de cellules.

10. Procédé selon l'une quelconque des revendications 1 à 8, dans lequel les cellules cultivées sur la matrice d'hydrogel en 3D appartiennent à au moins deux types de cellules.

11. Procédé selon l'une quelconque des revendications 1 à 10, dans lequel les cellules cultivées sur la matrice d'hydrogel en 3D appartiennent au groupe constitué de cellules souches, de cellules souches pluripotentes induites, de cellules progénitrices, de cellules différenciées, et de combinaisons de celles-ci.

12. Procédé selon l'une quelconque des revendications 1 à 11, dans lequel la matrice d'hydrogel en 3D est un hydrogel en 3D d'hyaluronane réticulé et l'enzyme utilisée dans l'étape (b) est une hyaluronidase.

13. Procédé selon la revendication 12, dans lequel, avant la culture cellulaire, l'hydrogel en 3D d'hyaluronane réticulé est :
(i) lyophilisé ; et
(ii) stérilisé
d'abord par chauffage de l'hydrogel, et
ensuite par immersion de l'hydrogel dans un alcool pur et soumission de l'hydrogel immergé à une compression physique.

14. Procédé selon l'une quelconque des revendications 1 à 13, dans lequel les cellules récoltées sont saines, viables, et totalement fonctionnelles.
